# EUROPÄISCHE PATENTSCHRIFT

(11) **EP 1 177 829 B1**
(45) Veröffentlichungstag und Bekanntmachung des Hinweises auf die Patenterteilung: **10.10.2007**
(21) Anmeldenummer: 01115145.3
(22) Anmeldetag: 22.06.2001
(51) Int. Cl.: B01D 67/00, B01D 69/02, B01D 71/42, A61L 27/16, C08L 33/20

(54) **Mit Blut und Gewebe verträgliche Membranen aus P(AN/NVP)-Misch-polymeren und deren Anwendung im medizinischen Bereich**
Blood and tissue compatible membranes from polyacrylonitrile/N-vinylpirrolidone copolymers and their use in the medical field
Membranes compatibles avec le sang et le tissu à base de copolymères d'acrylonitrile/N-vinylpyrrolidone et leur utilisation dans le domaine de la médecine

(30) Priorität: 27.06.2000 DE 10030307
(43) Veröffentlichungstag der Anmeldung: 06.02.2002
(73) Patentinhaber: GKSS-Forschungszentrum Geesthacht GmbH, 21502 Geesthacht (DE); University of Patras, 26500 Patras (GR)
(72) Erfinder: Groth, Dr. Thomas, 10439 Berlin (DE); Seifert, Dr. Barbara, 10249 Berlin (DE); Albrecht, Dr. Wolfgang, 14513 Teltow (DE); Malsch, Dr. Günter, 14513 Teltow (DE); Fey-Lamprecht, Frédérique Dr., 85521 Ottobrunn (DE); Gross, Ulrich Prof. Dr., 14195 Berlin (DE); Missirlis, Yannis Prof., 26500 Achaia (GR); Mihanetzis, Georgios Dr., 26500 Ovvia Patras (GR); Courtney, James M. Prof., Glasgow G66 4EA (GB); Grant, Helen Dr., Glasgow G61 3ET (GB); Engbers, Gerard Dr., 7577 MB Oldenzaal (NL)
(74) Vertreter: Seemann, Ralph

(56) Entgegenhaltungen:
- WO-A-95/25582
- DE-A- 4 341 601
- GB-A- 1 387 604
- US-A- 2 790 783
- US-A- 2 971 937
- DATABASE WPI Week 199051 Derwent Publications Ltd., London, GB; AN 1990-379397 XP002249948 & JP 02 273522 A (ASAHI MEDICAL CO LTD), 8. November 1990 (1990-11-08)
- DATABASE WPI Week 199910 Derwent Publications Ltd., London, GB; AN 1999-113838 XP002249949 & JP 10 337456 A (ASAHI KASEI KOGYO KK)
- F.FEY-LAMPRECHT ET AL: "development of membranes for the cultivation of kidney epithelial cells" BIOMATERIALS, Bd. 21, 2000, Seite 183-192 XP002219866

## Beschreibung

Die Erfindung betrifft eine Membran, die aus einem Mischpolymer hergestellt ist, sowie ein Verfahren zur Herstellung einer Membran aus einem Mischpolymer. Ferner betrifft die Erfindung eine Verwendung einer Membran.

Membranen mit mindestens einem Polyacrylnitril-Mischpolymer sind für biomedizinische Einsatzzwecke wie beispielsweise als Träger und für Trennzwecke in biohybriden Organen, als Oberflächenbeschichtungen für implantierbare Sensoren und für andere biomedizinische Anwendungsbereiche bestimmt.

Biohybride bzw. bioartifizielle Organe sind als Träger oder auch als vorübergehender Ersatz für funktionell gestörte menschliche Organe vdrgesehen. Das Hybrid besteht dabei aus Membranen mit "passiven" Trenn-, Filter- und Trägerfunktionen sowie aus lebenden Organzellen mit "aktiven" Transport- und Sekretionsfunktionen.

Bei bisher bekannten künstlichen bzw. artifiziellen Organen, beispielsweise der künstlichen Niere, wurden nur die passiven Trenn- und Filterfunktionen des Organs mit Hilfe spezialisierter polymerer Membranen ersetzt. In der Niere schließt sich aber an den Filtervorgang ein aktiver Prozess zur Readsorption an, bei welchem Bestandteile aus dem primären Urin und Wasser entgegen einem Konzentrationsgradienten vom primären Urin zurück ins Blut transportiert werden. Dieser aktive Prozess wird vom Epithel der Niere realisiert. Außerdem besitzt das Epithel der Niere eine endokrine Funktion, die für die Freisetzung von erythropoetischem Faktor, Vitamin D, Hormonen, Angiotensinen vom Typ II, Kininen und Reninen zuständig ist, welche zur Homöostase des Organismus beitragen.

Vergleichbare Situationen liegen bei der Leber vor, die eine der wichtigsten endokrinen Drüsen im Organismus ist und vor allem eine Entgiftungsaufgabe erfüllt. Es ist klar, dass mit Hilfe der Porenmembranen nur eine passive Filterfunktion und, was noch günstiger ist, eine Sorptionsfunktion der Organe, die für die Entgiftung zuständig sind (Leber, Niere), erzielt werden und somit zu Therapiezwecken eingesetzt werden kann.

Aus diesem Grund wurde angeregt, Organzellen in sogenannten biohybriden Organen zu immobilisieren, um sich die Funktion des aktiven Transports und der Sekretion lebensfähiger Zellen zunutze zu machen, mit dem Ziel, erkrankte Organe in ihren Funktionen zu unterstützen oder um diese Funktionen zu ersetzen. Bis heute sind die erzielten Ergebnisse zu Therapiezwecken nur in geringem Umfang einsetzbar, da
- einerseits die Lebensfähigkeit von Zellen nicht über den erforderlichen Zeitraum aufrecht erhalten werden kann, und
- andererseits die bisher erzielte Funktionalität der immobilisierten Zellen nicht der Funktionalität des natürlichen Organs entspricht.

Einer der wichtigsten Gründe für diese unbefriedigende Funktionalität ist darauf zurückzuführen, dass diese insofern ihren Ursprung in der Membran hat, als letztere ein Träger von Zellen ist, die von der Verankerung abhängig sind. Nach dem heutigen Wissensstand sollte der Membranträger die folgenden Eigenschaften besitzen, damit die Zellen eine optimale Funktionalität haben:
1. Im allgemeinen stehen primäre Zellen aus dem zu behandelnden Organ eines Patienten nicht in ausreichender Anzahl zur Verfügung. Deshalb müssen bei biohybriden Organen Spenderzellen menschlichen oder tierischen Ursprungs verwendet werden. In jedem Fall ist eine Abschirmung des Patienten gegenüber allogenen oder xenogenen Zellen im Bioreaktor erforderlich, um eine Aktivierung des Immunsystems beim Wirt zu verhindern. Diese Immunisolierung setzt der grösstmöglichen Porengrösse der Membran Grenzen. Antikörper, Komplementfaktoren, Viren usw. dürfen die Membran nicht passieren. Andererseits sollte die Permeabilität der Membran den Austausch der für die Versorgung (Nährstoffe, einige Proteine wie Albumin, Gase, zu entfernende Toxine) notwendigen Substanzen und der Zellprodukte (Stoffe aus dem Zellstoffwechsel, spezielle Proteine, usw.) zulassen.
2. Organzellen wie beispielsweise Leberzellen oder Zellen aus dem Epithel der Niere sind Zellen, die von Adhäsion abhängig sind und an den Stellen ihres Kontakts mit der Membran, an der sie verankert sind, eine Matrix außerhalb der Zellen voraussetzen. Nur auf diese Weise bleiben sie über einen längeren Zeitraum lebensfähig und entwickeln ihre normale Morphologie und Funktion und organisieren sich in polaren, gewebeähnlichen Strukturen. Somit muss die Membran die mäßige Adsorption von extrazellulären Matrixproteinen ohne größere Formveränderungen zulassen und deren spätere strukturelle Organisation ermöglichen. Infolgedessen muss die mit den Gewebezellen in Berührung kommende Membranfläche extreme Voraussetzungen erfüllen, um mit dem Gewebe kompatibel zu sein.
3. Zur Blutentgiftung und zum Ersatz von Organen gehört grundsätzlich der Kontakt des Vollblutes mit allen Zellen- und PlasmaBestandteilen bzw. dem Plasma mit allen seinen Proteinbestandteilen auf einer Seite der Membranfläche. Insbesondere muss im Falle von Biohybridsystemen ein Langzeitkontakt (mindestens über einige Tage oder Wochen) erwartet werden. Deshalb werden unter Umständen Blutproteine und Blutzellen nicht aktiviert, um eine Aktivierung der Koagulations-, Fibrinolyse-, Komplement- und Zellsysteme im Blut zu vermeiden. Die mit dem Blut bzw. dessen Bestandteilen in Berührung kommende Membranfläche muss somit extrem hohe Voraussetzungen erfüllen, um blutverträglich zu sein.
4. Je nach dem zu unterstützenden oder zu ersetzenden Organ und entsprechend den vorstehend in den Punkten 1 bis 3 genannten Voraussetzungen kommt der Fähigkeit der Membran zur Adsorption eine ausschlaggebende Bedeutung zu. Endokrine Organe produzieren Proteine, die ohne deutlichere Adsorption während des Durchtritts durch die Membranen in den Blutstrom eingebracht werden müssen, damit sie ihre Aufgabe im menschlichen Körper erfüllen können. Somit besitzt die Membran gegebenenfalls nur eine mäßige Adsorptionsfähigkeit gegenüber Proteinen.

In WO-A-95/25582 ist eine asymmetrische semipermeable Membran für die Dialyse und/oder Ultrafiltration beschrieben, die aus einem Copolymer aus Acrylnitril und Acrylester sowie einem ionischen oder ionisierbaren Monomer in der Form eines Hohlfadens mit einer Trennschicht im Inneren des Hohlfadens zusammengesetzt ist. Das Monomer ist zusammengesetzt aus 90,50 bis 95,00 Gew.-% Acrylnitril sowie 4,99 bis 8,60 Gew.-% Acrylester und 0,01 bis 0,90 Gew.-% des ionischen oder ionisierbaren Monomers. Die Membran enthält ferner bis zu 5% an Polyvinylpyrrolidon bezogen auf das Copolymer.

Darüber hinaus ist in DE-A-43 41 601 eine Membran aus Acrylnitril-Copolymeren beschrieben, die zu 60 bis 100% ihres Gesamtgewichts aus einem Acrylnitril-Copolymer A) und zu 40 bis 0% ihres Gesamtgewichts aus einem mit A) verträglichen Polymer B) besteht. Das Acrylnitril-Copolymer A) weist zudem folgende Zusammensetzung in Gew.-%, bezogen auf das Gesamtgewicht von A) auf: 70 bis 95% Acrylnitril, 0 bis 1 % Methallylsulfonsäure oder deren Alkalimetallsalze und 5 bis 30% eines Komonomeren aus der Gruppe der nicht ionischen Vinyl- und (Meth)Acrylsäure-Derivate. Das mit A) verträgliche Polymer weist zudem eines oder mehrere wasserlösliche Polymere aus der Gruppe von Polyvinylpyrrolidon sowie weitere Copolymerisate auf. Hierbei sind im Acrylnitril-Copolymer A) neben Acrylnitril stets Komonomere aus der Gruppe der nicht ionischen Vinyl- und (Meth)Acrylsäure-Derivate vorhanden.

In US-A-2 79.0 783 ist überdies die Herstellung von Fasern aus Acrylnitril-Polymeren beschrieben, die Polyvinylpyrrolidone aufweisen.

### Kurzbeschreibung der Erfindung

Der Erfindung liegt somit die Aufgabe zugrunde, eine Membran vorzuschlagen, die mit Blut und Gewebe verträglich und für biomedizinische Anwendung wie beispielsweise für Träger- und Trennzwecke bei biohybriden Organen als Ersatz für die Leber- und Nierenfunktion und auch als Oberflächenbeschichtung implantierbarer Biosensoren oder auf anderem implantierbaren Material geeignet ist.

Diese Aufgabe wird mit einer Membran nach den Merkmalen des Anspruchs 1 sowie einem Verfahren zur Herstellung einer Membran nach den Merkmalen des Anspruchs 6 gelöst.

Die erhaltenen Membranen mit einer Zusammensetzung eines Polymermaterials aus Acrylnitril (AN) und N-Vinylpyrrilidon (NVP) lassen sich hinsichtlich ihres Trennverhaltens so einstellen, dass sie für Nährstoffe, Toxine, Abfallprodukte und Proteine von geringem Molekulargewicht permeabel, d.h. durchlässig sind und die immobilisierten Organzellen gegenüber dem Immunsystem des Empfängers schützen. Die mit Blut, Plasma, Medium, usw. in Berührung stehende Membranfläche muss hämokompatibel bzw. blutverträglich sein, was eine nur mäßige oder vernachlässigbar geringe Aktivierung der Blutbestandteile wie die Koagulation des Komplementsystems und der Thrombozyten bedeutet. Die mit dem Gewebe in Berührung stehende Seite der Membran muss dabei mit Organzellen verträglich sein, was bedeutet, dass die Festsetzung, Morphologie, Wachstum und Funktion dieser Zellen jeweils den entsprechenden Parametern im natürlichen Gewebe so nahe wie möglich kommen.

Eine Eigenschaft der Erfindung besteht somit darin, die Mischpolymer-Zusammensetzung hinsichtlich ihres Molekulargewichts und der hydrophilierenden NVP-Molfraktion so maßzuschneidern, dass man ein Mischpolymer erhält, dessen Lösung sich durch einen Prozess mit Phasenumkehr an Membranen bilden lässt, welche die gewünschte Permeabilität (Porengröße und Porenverteilung) aufweisen, und das insbesondere eine hohe Verträglichkeit mit Blut und Gewebe aufweist.

Neben den guten Eigenschaften der P(AN/NVP)-Mischpolymeren zur Membranbildung wurde eine entsprechende Verträglichkeit mit Blut und Gewebe bei einer NVP-Molfraktion zwischen 5 und 30 Mol-%, insbesondere bei 15 bis 30 Mol-%, beim Mischpolymer festgestellt.

Erfindungsgemäß wird die Verwendung eines Materials mit ausgewogener Benetzbarkeit anhand der Mischpolymerisation von Acrylnitril mit einem hydrophilierenden Ko-Monomeren für Anwendungszwecke beschrieben, bei denen das Material sowohl mit Gewebezellen als auch mit Blut in Berührung steht. Die Erfindung zeichnet sich durch ein Material aus, bei dem es sich um ein Mischpolymer aus Acrylnitril und N-Vinylpyrrolidon (NVP) mit einer Molfraktion von 5 bis 30 Mol-% NVP handelt. Das Material wird vorzugsweise zur Bildung von Filmen und Membranen verwendet. Mit diesen Formen kann das Material mit Gewebe und Gewebezellen von einer Seite aus und mit Blut von der anderen Seite aus in Berührung kommen. Es ist auch möglich, dieses Mischpolymer zur Herstellung von Oberflächenbeschichtungen in anderen geometrischen Ausbildungen heranzuziehen, wie beispielsweise kommerziellen Membranen, Schäumen und Gerüsten für die Gewebetechnik, um permeable Oberflächenbeschichtungen für Biosensoren herzustellen, wenn ein hohes Maß an Verträglichkeit sowohl mit Gewebezellen als auch Blut gefordert ist. Insbesondere wird das Mischpolymer zur Bildung von Membranen mit einer durchschnittlichen Porengröße von 5 bis 12 nm in einem Prozeß mit Phasenumkehr eingesetzt. Die Porosität dieser Membranen läßt eine Wasserströmung durch die Membranen im Bereich zwischen 1 und 10 l/m²hkPa und eine Rückhaltung von 150 bis 1.000 kD zu. Die Erfindung zeichnet sich des weiteren durch Eigenschaften der Membranfläche auf, zu denen eine hohe Verträglichkeit mit Blut und Gewebe gehört. Dabei führt die Membran nur zu einer mäßigen bzw. vernachlässigbar geringen Aktivierung der Blutbestandteile und sorgt für eine gute Unterstützung beim Zellenwachstum zur Bildung gewebeähnlicher Strukturen, d.h. eines Pseudo-Epithels, und ein hohes Maß an Funktionalität. Außerdem zeichnet sich die Erfindung durch die Verwendung dieser Materialien und Membranen auf medizinischem Gebiet zur Bildung von biohybriden Organen, Biosensoren oder Implantaten im Körper aus, wo sie sowohl mit Blut als auch mit Gewebezellen konfrontiert werden.

### Kurzbeschreibung der Zeichnung

Nachstehend wird die Erfindung nun anhand von Beispielen für die Polymerzusammensetzung, das Trennverhalten der Membran und die Eigenschaften der Membranen im Kontakt mit Blut, Zellen aus der Epithel der Niere und Leberzellen unter Bezugnahme auf die beigefügte Zeichnung beschrieben, in deren Mikroskopbildern, Schemazeichnungen und Graphiken jeweils folgendes dargestellt ist:
- Fig. 1:: die Rückhaltung von Thrombozyten unter statischen Bedingungen nach 30-minütigem Kontakt mit Materialien (Mittelwert ± SD, zum Vergleich: an Thrombozyten reiches Plasma vor Kontakt mit dem Material);
- Fig. 2:: nicht-aktivierte partielle Thromboplastin-Zeit unter statischen Bedingungen nach 30-minütigem Kontakt mit Materialien (Mittelwert ± SD, zum Vergleich: an Thrombozyten armes Plasma ohne Kontakt mit dem Material);
- Fig. 3:: Aktivierung des Komplementsystems (Gehalt an Bb-Fragmenten in einem an Thrombozyten armen Plasma) unter statischen Bedingungen nach 15-minütigem Kontakt mit Materialien (Durchschnittswert ± SD);
- Fig. 4:: Anhaftung und Aktivierung von Thrombozyten, angegeben durch die P-Selektin-Expression unter dynamischen Bedingungen nach 30-minütigem Kontakt mit Materialien (Mittelwert ± SD);
- Fig. 5:: Wachstum von MDCK-Zellen nach 3- und 6-tägiger Kultur auf den Membranen, ausgehend von der Zellenanzahl hochgerechnet nach einem Lactatdehydrogenase-Test (LDH-Test) (Mittelwert ± SD);
- Fig. 6:: MDCK-Zellen nach 7-tägiger Kultur auf NVP5, Anfärbung mit Hämatoxylin und Eosin, mit einer Länge des Markierstäbchens von 50 µm;
- Fig. 7:: MDCK-Zellen nach 7-tägiger Kultur auf NVP20, TEM-Mikrographik, mit einer Länge des Markierstäbchens von 1 µm;
- Fig. 8:: MDCK-Zellen nach 7-tägiger Kultur auf NVP5, TEM-Mikrographie, mit einer Länge des Markierstäbchens von 4 µm;
- Fig. 9:: Entwicklung der Zellenanzahl, angegeben durch die Lactatdehydrogenase-Aktivität von LLC-PK1-Zellen nach 3- und 6-tägiger Anzüchtung auf unterschiedlichen Trägern (Mittelwert ± SD);
- Fig. 10:: LLC-PK1-Zellen nach 7-tägiger Anzüchtung auf NVP30, nach Anfärbung mit Hämatoxylin und Eosin, mit einer Länge des Markierstäbchens von 50 µm;
- Fig. 11:: LLC-PK1-Zellen nach 7-tägiger Anzüchtung auf NVP20, TEM-Mikrographie, mit einer Länge des Markierstäbchens von 40 µm.

### Beispiele:

### Beispiel 1: Charakterisierung der Polymeren

Die Polymeren wurden aus Acrylnitril (AN) und N-Vinylpyrrilidon (NVP) synthetisiert und nach der Mischpolymer-Zusammensetzung durch Elementaranalyse und nach zahlenmäßigem durchschnittlichem Molekulargewicht durch Membran-Osmometrie (Tabelle 1) charakterisiert.

**Tabelle 1: Kennwerte der Polymeren**

| Polymerisationsmuster | Kurzbezeichnung | Kennwerte der Polymeren | |
|---|---|---|---|
| | | X_{Com, Pol} (Mol-%) | M_{n,OBm} (g/Mol)x10⁻³ |
| PAN | PAN | - | 48,5 |
| P(AN/NVP) 5 | NVP5 | 4,8 | 38,8 |
| P(AN/NVP) 20 | NVP20 | 20,9 | 37,5 |
| P(AN/NVP) 30 | NVP30 | 31,1 | 105,0 |
| P(AN/NaMAS | NaMAS | 1,9 | 10,6 |

| | | | |
|---|---|---|---|
| x_{Com}, _{Pol} = Komonomer-Konzentration im Mischpolymer M_{n,OBm} = Molekulargewicht der Mischpolymeren | | | |

PAN und P(AN/NaMAS) werden dabei als Vergleichsmaterial herangezogen. Die in der Beschreibung und der Kurzbezeichnung des NVP-Materials angegebene Zahl bezeichnet dabei den Wert der Mol-% an NVP im entsprechenden Mischpolymeren.

### Beispiel 2: Membranbildung

In einem Prozeß mit Phasenumkehr wurden Membranen aus einer Lösung der Polymeren nach Beispiel 1 in Dimethylformamid hergestellt. Die Polymerkonzentration wurde zwischen 15 und 25 Gew.-% verändert. Die Lösung wurde auf einen Vliesträger oder direkt auf einen Edelstahlstreifen mit einem Gießschlitz von 150 bzw. 300 µm aufgegossen, wobei die Ziehgeschwindigkeit zwischen 2 und 4 m/min betrug. Nach Durchlauf durch ein Wasser-Ausfällbad bei 5 °C bzw. Zimmertemperatur wurden einige der Membranen zehn Minuten lang bei 65 °C bzw. 90°C wärmebehandelt.

Bei den Beispielen 4 bis 6 wurden Membranen auf einer Vliesunterlage mit den nachstehenden Daten zur Herstellung gebildet (Tabelle 2).

**Tabelle 2: Daten zur Membranbildung bei den Beispielen 4 bis 6 (Ziehgeschwindigkeit: 4 m/Min; Fällungsbad: 5 °C)**

| Membran | Polymerkonzentration in Lösung (Gew.-%) | Temperatur der Polymerlösung | Breite des Gießschlitzes | Wärmebehandlung |
|---|---|---|---|---|
| PAN | 15 | 1 - 2°C | 300 µm | 10 Min, 90°C |
| NVP5 | 20 | 1 - 2°C | 300 µm | 10 Min, 90°C |
| NVP20 | 25 | 1 - 2°C | 150 µm | 10 Min, 65°C |
| NVP30 | 22,5 | Raumtemp. | 150 µm | 10 Min, 65°C |
| NaMAS | 25 | 1 - 2 °C | 300 µm | 10 Min, 90°C |

Bei diesen Membranen wurden die folgenden Werte für die Wasserdurchlässigkeit und den Wasserkontaktwinkel gemessen (Tabelle 3). Die Mischpolymerisation von AN mit NVP und NaMAS erbrachte einen Anstieg in der Wasserdurchlässigkeit. Bei den NVP-Mischpolymeren wurden nur geringfügige Veränderungen im Wasserkontaktwinkel (Benetzung) in Vorwärtsrichtung gemessen, wohingegen die Mischpolymerisation mit NaMAS kleinere Wasserkontaktwinkel in Vorwärtsrichtung erbrachte, was ein Hinweis auf eine Oberfläche mit stärkerer Wasserbindung ist.

**Tabelle 3: Wasserdurchlässigkeit und Wasserkontaktwinkel bei den Membranen aus den Beispielen 4 bis 6**

| Membran | Wasserströmung [l/m²hkPa] | Wasserkontaktwinkel | |
|---|---|---|---|
| | | vorwärts [°] | rückwärts [°] |
| PAN | 4,02 | 59,5 | 43,0 |
| NVP5 | 0,27 | 58,1 | 34,3 |
| NVP20 | 0,001 | 58,5 | 35,2 |
| NVP30 | - | 64,0 | 38,8 |
| NaMAS | 0,22 | 17,8 | 15,8 |

### Beispiel 3: Siebverhalten der Membranen

NVP20 wurde für den Nachweis der Fähigkeit zur selektiven Veränderung der Porosität und des Weiterleitungsverhaltens während des Prozesses zur Membranbildung gewählt.

Es wurden Membranen aus Mischpolymeren gemäß Beispiel 1 und 2 aus einer Gießlösung mit 15 bis 22,5 Gew.-% gebildet. Die Wasserdurchlässigkeit und das Siebverhalten von Membranen wurde in einer Vorrichtung zur Ultrafiltration - einer Berghoff-Zelle mit Rührwerk - mit destilliertem Wasser und einer Lösung aus einem Gemisch mit der Testsubstanz (Dextrane mit einem Molekulargewicht zwischen 650 und 215.000) bestimmt. Die Verteilungen der Dextrane nach Molekülgröße in den Permeaten, den zurückgehaltenen Substanzen und in der ursprünglichen Lösung wurden unter Einsatz der Gel-Permeations-Chromatographie (GPC) bestimmt. Anhand der Ergebnisse aus der GPC-Untersuchung wurden der durchschnittliche Porendurchmesser (D₅₀), der größtmögliche Porendurchmesser (D₁₀₀) und das entsprechende jeweilige Molekulargewicht unter Ansatz einer logarithmischen normalen Verteilung der Porengröße berechnet (Tabelle 4).

**Tabelle 4: Trennfähigkeit und Leistungsmerkmale bei Membranen aus AN/NVP20-Mischpolymeren**

| Membran | C_{Mischpolymer} [Gew.-%] | Wasserströmung [l/m²hkPa] | Porenmerkmale | | | |
|---|---|---|---|---|---|---|
| | | | D₅₀ [nm] | D₁₀₀ [nm] | σ | Rückhaltung [kD] |
| 22,5 NVP20 | 22,5 | 0,709 | 5,55 | 18,9 | 0,4250 | 164 |
| 20 NVP20 | 20 | 1,29 | 6,83 | 26,15 | 0,4656 | 338 |
| 19 NVP20 | 19 | 1,82 | 6,62 | 27,64 | 0,4958 | 382 |
| 18 NVP20 | 18 | 6,46 | 10,08 | 40,60 | 0,4831 | 897 |
| 15 NVP20 | 15 | 10,36 | 11,90 | 42,02 | 0,4376 | 968 |

Die Trenn-Parameter bei 19 NVP20 erfüllen die Anforderungen an eine Membran zur Immunisolierung (Rückhaltung 300 bis 400 kD) sowie zur Zuführung von Nährstoffen und zur Ableitung von Abfallstoffen bei Gewebezellen (Wasserströmung etwa 2 I/m²hkPa).

### Beispiel 4: Membranen in Kontakt mit Blut

Die Membranen gemäß den Beispielen 1 bis 2 wurden auf Verträglichkeit mit Blut unter statischen Bedingungen in der Form untersucht, daß sie einfach mit an Thrombozyten armem oder reichem menschlichen Blut in einer Teflonkammer mit 1 ml Plasma pro Vertiefung (22 mm Durchmesser) geschüttelt wurden. Mit einer Strömungskammer mit parallelen Platten und einem Kapillar-Perfusionsmodell wurden jeweils mit an Thrombozyten reichem menschlichen Plasma dynamische Bedingungen realisiert.

### 4.1 Statische Bedingungen

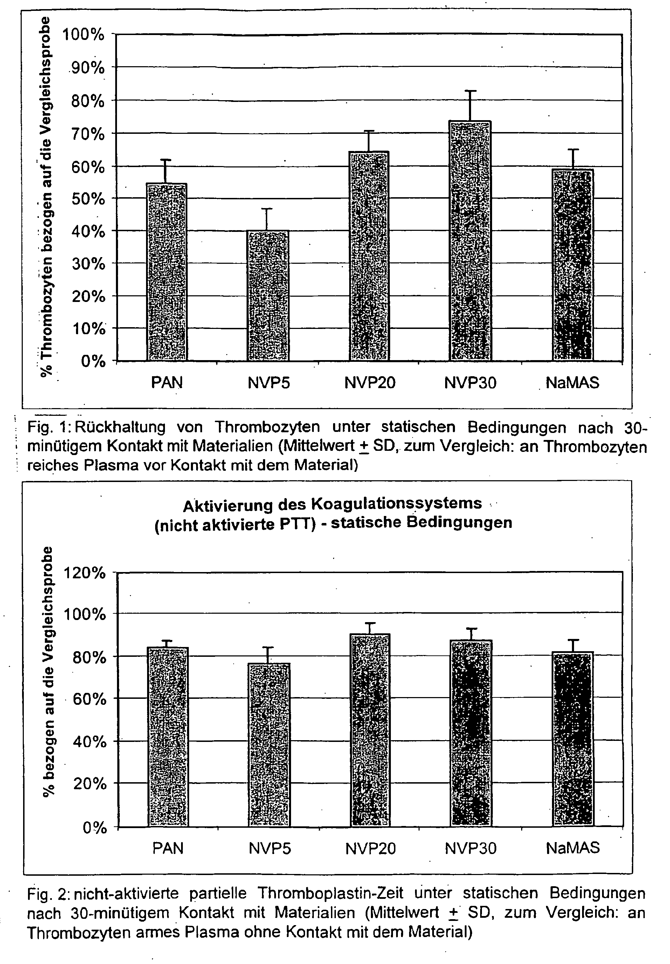

Unter statischen Bedingungen wurde die Rückhaltung von Thrombozyten nach 30-minütigem Kontakt eines an Thrombozyten reichen Plasmas mit flachen Membranen durch Thrombozytenzählung gemessen (Fig. 1). Es tritt ein Anstieg der zurückgehaltenen Thrombozyten auf, was bedeutet, daß nicht anhaftende oder nicht zusammengeballte Thrombozyten vorhanden sind, bei einem Anstieg des NVP-Gehalts auf bis zu 30 Mol-%. Die Vergleichsmaterialien PAN und NaMAS hielten weniger Thrombozyten als NVP20 und NVP30 zurück.

Die sogenannte nAPTT-Zeit, also die Zeit zur partiellen Bildung von nicht aktiviertem Thromboplastin, liefert einen Hinweis auf die Aktivierung des Koagulationssystems. Damit wird der eigene Koagulationsweg (Faktoren XII, XI, IX, VIII, X, V, II und I) untersucht. Nach 30-minütigem Kontakt des an Thrombozyten armen menschlichen Plasmas mit den Membranen erfuhr der nAPTT-Wert bei NVP20 und NVP30 eine geringere Veränderung, während die Koagulationszeit nur wenig verkürzt war (Fig. 2).

Bei der Aktivierung des Komplementsystems, die durch die Konzentration des Bb-Fragments nach 15-minütigem Kontakt des an Thrombozyten armen Plasmas angezeigt wird, trat bei NVP20 unter allen NVP-Materialien der niedrigste Wert auf.

### 4.2 Dynamische Bedingungen

Zur Untersuchung unter dynamischen Bedingungen wurden die Membranen in einer rohrähnlichen Form mit einer Länge von 25 cm und einem Durchmesser von 3 mm eingesetzt. Das an Thrombozyten reiche Plasma wurde durch dieses Röhrchen mit einer Schergeschwindigkeit von 30/Sek. 30 Minuten lang perfundiert.

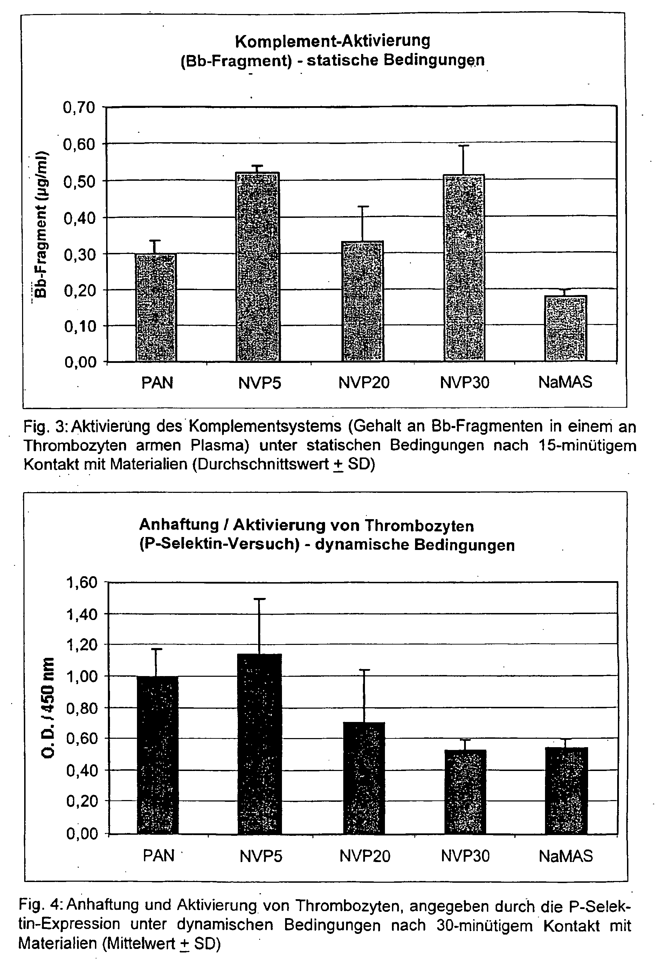

Unter dynamischen Bedingungen haften im Vergleich zu statischen Bedingungen weniger Thrombozyten auf allen Membranen an. Die Anhaftung und Aktivierung der Thrombozyten, die durch eine P-Selektin-Expression auf den Thrombozyten angezeigt wird, wurde durch einen ELISA-Test für P-Selektin (CD62, Fig. 4) nachgewiesen. Mischpolymere mit höheren NVP-Molverhältnissen (20 und 30 Mol-%) senkten die Anhaftung und Aktivierung der Thrombozyten erheblich.

Als Schlußfolgerung aus Beispiel 4 läßt sich feststellen, daß Membranen, die aus PAN-Mischpolymeren mit einem NVP-Gehalt von mehr als 5 Mol-% gefertigt sind, eine gute Verträglichkeit mit Blut aufweisen, die recht ähnlich der Verträglichkeit von NaMAS ist, das auch als Material für Dialysemembranen mit der Bezeichnung AN 69S bekannt ist.

### Beispiel 5: Membranen in Kontakt mit Epithelzellen aus der Niere

Die Membranen nach den Beispielen 1 bis 2 wurden hinsichtlich ihres Kontakts mit Epithelzellen aus der Niere untersucht. Dieses Beispiel wurde mit zwei Typen von Nierenzell-Linien durchgeführt, und zwar mit MDCK-Zellen (distale röhrenförmige Nierenzellen vom Hund nach Madin Darby) und LLC-PK1-Zellen (proximale röhrenförmige Nierenzellen vom Schwein).

Eine Kombination aus den beiden Zell-Linien repräsentiert das vollständige Gefäßsystem in der Niere, das die durch die asymmetrischen Membranen einer künstlichen Niere simulierte glomeruläre Filtration ergänzen könnte. Die Impfung mit Zellen erfolgte in einer Dichte von 8 x 10⁴ Zellen/cm², woraufhin die Zellen bis zu 7 Tage lang bei 37 °C und 5 % CO₂ inkubiert wurden.

### 5.1: Untersuchungen an MDCK-Zellen

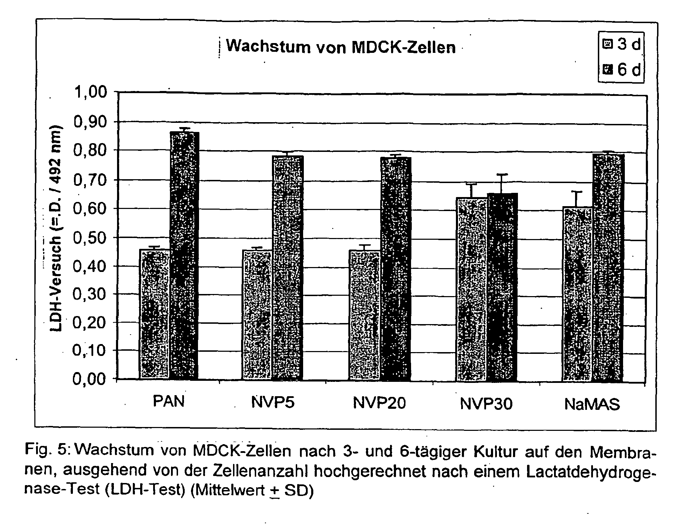

Der LDH-Versuch, der auf der Aktivität der Zytosol-Laktatdehydrogenase (proportional zur Anzahl lebensfähiger Zellen) aufbaut, weist eine zunehmende Anzahl von Zellen auf allen Membranen in Kulturen über einen Zeitraum zwischen 3 und 6 Tagen auf (Fig. 5), was auf gute Vorbedingungen für die Absetzung und das Wachstum von Zellen auf allen Membranflächen hinweist.

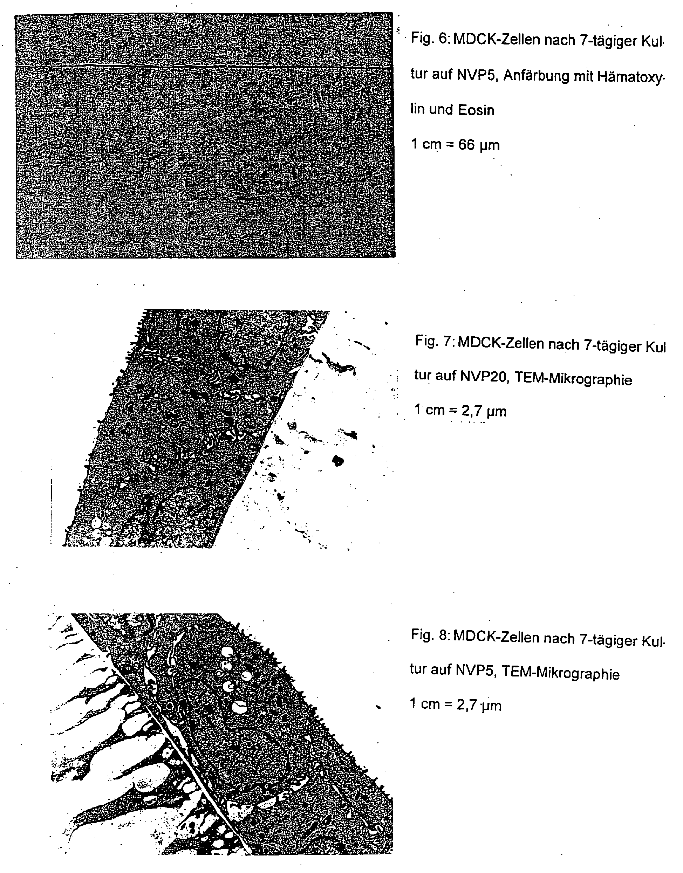

Zum Nachweis, ob eine vollständige Bedeckung der Membranoberfläche mit einer Zellschicht gegeben ist, wurde die Morphologie der Zellen durch ein herkömmliches Anfärbeverfahren unter Verwendung von Haematoxylin und Eosin sichtbar gemacht, wobei die Kerne blau bis purpurfarben und das Zytoplasma roséfarben gefärbt werden. Nach 7-tägiger Anzüchtung auf allen NVP- und PAN-Materialien läßt sich eine zusammenfließende Zellschicht beobachten (vgl. NVP5 in Fig. 6). Im Unterschied hierzu konnten MDCK-Zellen auf dem anderen Vergleichsmaterial, d.h. NaMAS, keine zusammenfließende und gleichmäßige Schicht bilden.

Dies wurde als weitere Voraussetzung für die Anwendbarkeit der NVP-haltigen Mischpolymere angesehen, da die Bildung einer Epithel-Filmschicht eine unabdingbare Voraussetzung für die Abtrennung verschiedener Fächer bzw. Kammern in einem Biohybridorgan darstellt.

Morphologie und Funktion der Zellen stehen in enger Beziehung zueinander. Deshalb wurde mit Transmissionselektronenmikroskopie (TEM) gearbeitet, um nachzuweisen, ob bei allen Zellen die Expression ihrer typischen Morphologie gegeben war. Bei allen NVP-Membranen zeigten MDCK-Zellen nach 7-tägiger Kultur ihre typischen Merkmale wie enge Zellkontakte und Mikrozotten (vgl. NVP20 in Fig. 7). Zum Vergleich zeigten NaMAS-Zellen weniger Mikrozotten, was als Hinweis auf eine reduzierte Funktionalität der Zellen gilt.

Die Funktionalität der MDCK-Zellen wurde durch Bestimmung des transepithelialen Widerstands unter Verwendung der Ussing-Kammer gemessen. MDCK-Zellen sollten einen Wert von etwa 1.000 bis 3.000 Ωm² zeigen. Der gemessene Widerstand ist direkt mit der Durchleitung von Na⁺- und K⁺-lonen durch die Zellschicht verknüpft. In einem normalen Epithel wird diese Durchleitung durch Öffnen und Schließen der engen Verbindungen zwischen den Zellen gesteuert. Die Werte für den transepithelialen Widerstand von MDCK-Zellen auf unterschiedlichen Trägern sind in Tabelle 5 angegeben. Bei den MDCK-Zellen zeigte sich auf NVP-Membranen ein höherer Wert für den Widerstand durch das Epithel als bei der Vergleichsmembran PAN, was ein Hinweis auf eine gut entwikkelte und in ihrer Funktionalität intakte Epithelschicht ist.

**Tabelle 5: Transepithelialer Widerstand bei MDCK-Zellen nach 7-tägiger Anzucht auf unterschiedlichen Trägern**

| **Membran** | **transepithelialer Widerstand** [Ωcm²] | |
|---|---|---|
| | Mittelwert | Standardabweichung |
| **PAN** | 1333 | 175 |
| **NVP5** | 1958 | 597 |
| **NVP20** | 2310 | 410 |
| **NVP30** | 8972 | 1672 |

### 5.2: Untersuchungen an LLC-PK1-Zellen

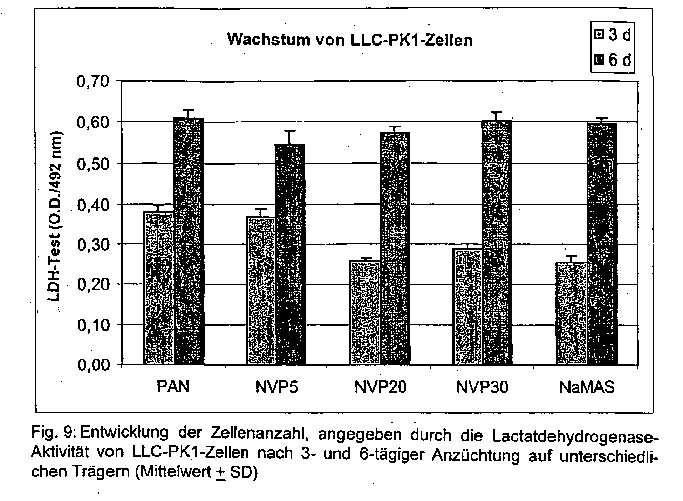

Der LDH-Versuch, der auf der Aktivität der Zytosol-Laktatdehydrogenase (proportional zur Anzahl lebensfähiger Zellen) aufbaut, weist eine zunehmende Anzahl von Zellen auf allen Membranen in Kulturen über einen Zeitraum zwischen 3 und 6 Tagen auf (Fig. 9), was auf gute Vorbedingungen für die Absetzung und das Wachstum von Zellen auf allen Membranflächen sowie die anschließende Bildung einer Epithelschicht mit der angestrebten Funktionalität hinweist.

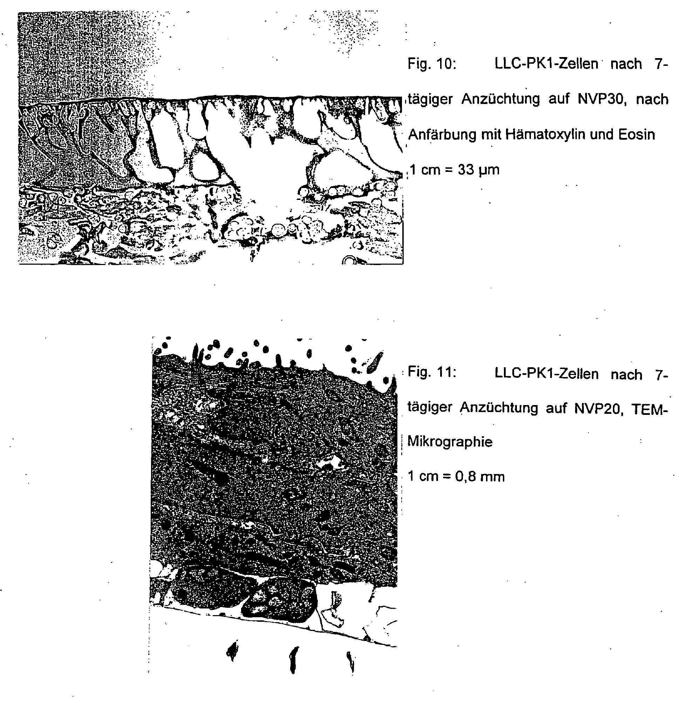

Untersuchungen zur Morphologie unter Anfärbung mit Hämatoxylin und Eosin in der vorbeschriebenen Weise lassen erkennen, daß auf allen Membranen zusammenfließende Schichten gut entwickelter Zellen vorhanden sind. LLC-PK1-Zellen nach 7-tägiger Kultur auf dem NVP30-Träger sind in Figur 10 dargestellt.

Bei Einsatz der Transmissionselektronenmikroskopie (TEM) lassen sich gut entwickelte Zellen auf allen Membranen beobachten (Mikrozotten und enge Zellverbindungen sind vorhanden). LLC-PK1-Zellen nach 7-tägiger Kultur auf NVP20 sind in Figur 11 dargestellt.

Die Funktionalität der LLC-PK1-Zellen wurde durch Bestimmung des transepithelialen Widerstands unter Verwendung der Ussing-Kammer gemessen.

LLC-PK1-Zellen sollten einen Wert von etwa 500 bis 1.000 Ωcm² zeigen. Der gemessene Widerstand ist direkt mit der Durchleitung von Na⁺- und K⁺-lonen durch die Zellschicht verknüpft. Bei den LLC-PK1-Zellen wurden Werte für den transepithelialen Widerstand gemessen, die in Tabelle 6 zusammengefaßt sind. Bei NVP5 ergeben sich Ergebnisse, die mit denen der Vergleichsmembran PAN vergleichbar sind. Bei NVP20 und NVP30 ergibt sich ein höherer transepithelialer Widerstand, der als Hinweis auf eine gut entwickelte und in ihrer Funktionalität intakte Epithelschicht gilt.

**Tabelle 6: Transepithelialer Widerstand bei LLC-PK1-Zellen nach 7-tägiger Anzucht auf unterschiedlichen Trägern**

| **Membran** | **transepithelialer Widerstand** [Ωm²] | |
|---|---|---|
| | Mittelwert | Standardabweichung |
| **PAN** | 334 | 165 |
| **NVP5** | 367 | 242 |
| **NVP20** | 767 | 132 |
| **NVP30** | 682 | 248 |

### Beispiel 6: Membranen in Kontakt mit Hepatozyten

Die biologische Verträglichkeit von Membranen nach den Beispielen 1 bis 2 als Träger von Primärkulturen von Hepatozyten von der Ratte wurde durch Messung des Testosteron-Stoffwechsels bewertet.

Hierzu erfolgte eine Impfung mit primären Hepatozyten von der Ratte in einer Dichte von 1,6 x 106 Zellen pro Membran (Kulturen auf Membranscheiben, 40 mm im Durchmesser) und 3 x 106 Zellen pro Petri-Schale, 60 mm Durchmesser, mit Kollagenbeschichtung für die Vergleichsprobe. Die Zellen wurden mit 5-%igem fötalem Kalbsserum in einem Medium nach Chee angezüchtet. Nach 48 Stunden in der Kultur wurden die Zellen auf den Membranen und die Vergleichsprobe mit 100 µM Testosteron in serumfreiem Medium 60 Minuten lang inkubiert, woraufhin die Produkte mit HPLC analysiert wurden. Tabelle 7 zeigt zwar, daß der höchste Wert für den Testosteron-Stoffwechsel bei Leberzellen auf einer NVP20-Membran festzustellen ist, doch ist die Funktionalität der Hepatozyten von der Ratte auf den Membranen geringer als bei der Vergleichsprobe auf Kollagen.

**Tabelle 7: Testosteron-Stoffwechselprodukte insgesamt, die von Zellkulturen auf den Membranen gebildet wurden (Vergleichsversuch = mit Kollagen beschichtetes Glas)**

| **Membran** | **Testosteron-Stoffwechsel insgesamt** [nmol/h/mg Protein] | |
|---|---|---|
| | Mittelwert | Standardabweichung |
| **Vergleichsprobe** | 5,35 | 0,19 |
| **PAN** | 1,50 | 1,50 |
| **NVP5** | 2,07 | 1,03 |
| **NVP20** | 2,77 | 1,88 |
| **NVP30** | 1,70 | 1,70 |
| **NaMAS** | 1,73 | 0,08 |

## Patentansprüche

1. Membran, die hergestellt ist aus einem Mischpolymer, das aus 70 bis 95 Mol-% Acrylnitril-Monomereinheiten und aus N-Vinylpyrrolidon mit einer Molfraktion zwischen 5 Mol-% und 30 Mol-%, insbesondere zwischen 15 Mol-% und 30 Mol-%, als nicht-elektrolytischen wasserlöslichen Komonomereinheiten besteht.

2. Membran nach Anspruch 1, **dadurch gekennzeichnet, dass** die Membran eine asymmetrische Membran ist, welche eine dichte Schicht mit einer durchschnittlichen Porengröße von 5 bis 12 nm als eine äußere Schicht aufweist.

3. Membran nach einem der Ansprüche 1 bis 2, **dadurch gekennzeichnet, dass** die Membran eine Membran mit flachen oder hohlen Fasern ist, welche eine zweischichtige Querschnittsstruktur aufweist, die im wesentlichen aus einer dichten Oberflächenschicht und einer porösen Füllschicht mit fingerähnlichen Poren besteht, die mit der dichten Schicht in Verbindung steht.

4. Membran nach einem der Ansprüche 1 bis 3, **dadurch gekennzeichnet, dass** bei der Membran die Geschwindigkeit der Wasserströmung durch die Membran im Bereich zwischen 1 und 10 l/m²hkPa liegt.

5. Membran nach einem der Ansprüche 1 bis 4, **dadurch gekennzeichnet, dass** bei der Membran die Rückhaltung im Bereich zwischen 150 und 1.000 kD liegt.

6. Verfahren zur Herstellung einer Membran aus einem Mischpolymer, das aus 70 bis 95 Mol-% Acrylnitril-Monomereinheiten und aus N-Vinylpyrrolidon mit einer Molfraktion zwischen 5 Mol-% und 30 Mol-%, insbesondere zwischen 15 Mol-% und 30 Mol-%, als nicht-elektrolytischen wasserlöslichen Komonomereinheiten besteht,
- Herstellen einer Gießlösung mit einer Konzentration an festen Mischpolymeren von 15 bis 25 Gew.-% durch Auflösen des Polymeren in einem polaren Lösungsmittel;
- Aufgießen der Lösung auf einen Träger oder Extrudieren der Lösung durch eine Düse, die ein Röhrchen in der Öffnung aufweist;
- Koagulieren der vergossenen bzw. extrudierten Lösung in einem Koagulierbad in einem Naßverfahren oder Naß-Trocken-Naß-Verfahren zur Bildung einer asymmetrischen Membran, und
- Vornahme einer Nachbehandlung im Nassen an der asymmetrischen Membran in Wasser oder Dampf.

7. Verfahren nach Anspruch 6, **dadurch gekennzeichnet, dass** die Membran durch einen Prozess mit Phasenumkehr gebildet wird.

8. Verfahren nach Anspruch 6 oder 7, **dadurch gekennzeichnet, dass** die Membran eine asymmetrische Membran ist, welche eine dichte Schicht mit einer durchschnittlichen Porengröße von 5 bis 12 nm als eine äußere Schicht aufweist.

9. Verfahren nach einem der Ansprüche 6 bis 8, **dadurch gekennzeichnet, dass** die Membran eine Membran mit flachen oder hohlen Fasern ist, welche eine zweischichtige Querschnittsstruktur aufweist, die im wesentlichen aus einer dichten Oberflächenschicht und einer porösen Füllschicht mit fingerähnlichen Poren besteht, die mit der dichten Schicht in Verbindung steht.

10. Verfahren nach einem der Ansprüche 6 bis 9, **dadurch gekennzeichnet, dass** bei der Membran die Geschwindigkeit der Wasserströmung durch die Membran im Bereich zwischen 1 und 10 l/m²hkPa liegt.

11. Verfahren nach einem der Ansprüche 6 bis 10, **dadurch gekennzeichnet, dass** bei der Membran die Rückhaltung im Bereich zwischen 150 und 1.000 kD liegt.

12. Verwendung einer Membran nach einem der Ansprüche 1 bis 5 oder einer Membran, die nach einem Verfahren gemäß einem der Ansprüche 6 bis 11 hergestellt ist, in biohybriden und bioartifiziellen Organen als Membran mit Filter- und Trägerfunktion für immobilisierte Organzellen wie Zellen aus der Niere, der Leber, der Bauchspeicheldrüse und anderen Organen, die aus Blut oder Blutbestandteilen abzutrennen sind.

13. Verwendung einer Membran nach einem der Ansprüche 1 bis 5 oder einer Membran, die nach einem Verfahren gemäß einem der Ansprüche 6 bis 11 hergestellt ist, oder eines Mischpolymers, das aus 70 bis 95 Mol-% Acrylnitril-Monomereinheiten und aus N-Vinylpyrrolidon mit einer Molfraktion zwischen 5 Mol-% und 30 Mol-%, insbesondere zwischen 15 Mol-% und 30 Mol-%, als nicht-elektrolytischen wasserlöslichen Komonomereinheiten besteht, für medizinische Anwendungen im Körper, bei denen sie sowohl mit Blut als auch dem umgebenden Gewebe konfrontiert sind.

## Claims

1. Membrane which is produced from a mixed polymer which consists of 70 to 95 mol.% acrylonitrile monomer units and of N-vinylpirrolidone with a mole fraction between 5 mol.% and 30 mol.% as non-electrolytic watersoluble comonomer units.

2. Membrane according to claim 1, **characterised in that** the membrane is an asymmetrical membrane which comprises a dense layer with an average pore size of 5 to 12 nm as an outer layer.

3. Membrane according to one of the claims 1 to 2, **characterised in that** the membrane is a membrane with flat or hollow fibres which has a two-layer cross-sectional structure which consists essentially of a dense surface layer and a porous fill layer with finger-like pores which is connected to the dense layer.

4. Membrane according to one of the claims 1 to 3, **characterised in that** the speed of water flow through the membrane lies in the range between 1 and 10 l/m²hkPa.

5. Membrane according to one of the claims 1 to 4, **characterised in that** the retention of the membrane lies in the range between 150 and 1000 kD.

6. Method for producing a membrane from a mixed polymer which consists of 70 to 95 mol.% acrylonitrile monomer units and of N-vinylpirrolidone with a mole fraction between 5 mol.% and 30 mol.%, in particular between 15 mol.% and 30 mol.% as non-electrolytic watersoluble comonomer units,
- production of a pouring solution with a concentration of solid mixed polymers of 15 to 20 wt.% by dissolving the polymer in a polar solvent;
- pouring of the solution onto a carrier or extrusion of the solution through a nozzle which comprises a tube in the opening;
- coagulation of the poured or extruded solution in a coagulation bath in a wet process or wet-dry-wet process in order to form an asymmetrical membrane, and
- carrying out curing in wet conditions on the asymmetrical membrane in water or steam.

7. Method according to claim 6, **characterised in that** the membrane is formed by a process with phase inversion.

8. Method according to claim 6 or 7, **characterised in that** the membrane is an asymmetrical membrane which comprises a dense layer with an average pore size of 5 to 12 nm as an outer layer.

9. Method according to one of the claims 6 to 8, **characterised in that** the membrane is a membrane with flat or hollow fibres which has a two-layer cross-sectional structure which consists essentially of a dense surface layer and a porous fill layer with finger-like pores which are connected to the dense layer.

10. Method according to one of the claims 6 to 9, **characterised in that** the speed of the water flow through the membrane lies in the range between 1 and 10 l/m²hkPa.

11. Method according to one of the claims 6 to 10, **characterised in that** the retention of the membrane lies in the range between 150 and 1000 kD.

12. Use of a membrane according to one of the claims 1 to 5 or a membrane which is produced according to a method according to one of the claims 6 to 11 in biohybrid and bioartificial organs as a membrane with filter and carrier functions for immobilised organ cells such as cells from the kidney, the liver, the pancreas and other organs which are to be separated out of blood or blood components.

13. Use of a membrane according to one of the claims 1 to 5 or a membrane which is produced according to a method according to one of the claims 6 to 11 or a mixed polymer which consists of 70 to 95 mol.% acrylonitrile monomer units and of N-vinylpirrolidone with a mole fraction between 5 mol.% and 30 mol.%, in particular between 15 mol.% and 30 mol.% as non-electrolytic watersoluble comonomer units, for medical applications in the body, in which they are confronted both with blood and also the surrounding tissue.

## Revendications

1. Membrane fabriquée à partir d'un polymère mixte qui se compose de 70 à 95 % en moles de motifs de monomère d'acrylonitrile et de N-vinylpyrrolidone avec une fraction molaire entre 5 % en moles et 30 % en moles, en particulier entre 15 % en moles et 30 % en moles, à titre de motifs de comonomère hydrosoluble non électrolytique.

2. Membrane selon la revendication 1, **caractérisée en ce que** la membrane est une membrane asymétrique, qui présente une couche dense ayant une taille de pores moyenne de 5 à 12 nm à titre de couche extérieure.

3. Membrane selon l'une des revendications 1 à 2, **caractérisée en ce que** la membrane est une membrane avec des fibres plates ou creuses, qui présente une structure transversale bicouche, qui se compose sensiblement d'une couche superficielle dense et d'une couche de remplissage poreuse avec des pores de type digital, qui est en relation avec la couche dense.

4. Membrane selon l'une des revendications 1 à 3, **caractérisée en ce que**, pour la membrane, la vitesse de circulation de l'eau à travers la membrane est dans la plage entre 1 et 10 litres/m² hkPa.

5. Membrane selon l'une des revendications 1 à 4, **caractérisée en ce que**, pour la membrane, la rétention se situe dans la plage entre 150 et 1000 kD.

6. Procédé de fabrication d'une membrane constituée d'un polymère mixte qui se compose de 70 à 95 % en moles de motifs de monomère d'acrylonitrile et de N-vinylpyrrolidone avec une fraction molaire entre 5 % en moles et 30 % en moles, en particulier entre 15 % en moles et 30 % en moles, à titre de motifs de comonomère hydrosoluble non électrolytique, consistant à :
- fabriquer une solution à verser ayant une concentration en polymère mixte solide de 15 à 25 % en poids par dissolution du polymère dans un solvant polaire ;
- verser la solution sur un support ou extruder la solution à travers une filière qui présente un petit tube dans l'ouverture ;
- faire coaguler la solution versée ou extrudée dans un bain de coagulation au cours d'un procédé par voie humide ou d'un procédé par voie humide - sèche - humide pour former une membrane asymétrique, et
- opérer un traitement ultérieur par voie humide sur la membrane asymétrique dans de l'eau ou à la vapeur.

7. Procédé selon la revendication 6, **caractérisé en ce que** la membrane est formée par un procédé comportant un inversement de phase.

8. Procédé selon la revendication 6 ou 7, **caractérisé en ce que** la membrane est une membrane asymétrique, qui présente une couche dense ayant une taille de pores moyenne de 5 à 12 nm à titre de couche extérieure.

9. Procédé selon l'une des revendications 6 à 8, **caractérisé en ce que** la membrane est une membrane ayant des fibres plates ou creuses, qui présente une structure transversale bicouche, qui se compose sensiblement d'une couche superficielle dense et d'une couche de remplissage poreuse avec des pores de type digital, qui est en relation avec la couche dense.

10. Procédé selon l'une des revendications 6 à 9, **caractérisé en ce que**, pour la membrane, la vitesse de circulation de l'eau à travers la membrane est dans la plage entre 1 et 10 litres/m² hkPa.

11. Procédé selon l'une des revendications 6 à 10, **caractérisé en ce que**, pour la membrane, la rétention se situe dans la plage entre 150 et 1000 kD.

12. Utilisation d'une membrane selon l'une des revendications 1 à 5 ou d'une membrane qui est fabriquée d'après un procédé selon l'une des revendications 6 à 11 dans des organes biohybrides et bioartificiels comme membrane ayant une fonction filtrante et porteuse pour des cellules d'organes immobilisées, telles que des cellules de reins, de foie, de pancréas et d'autres organes, qui doivent être séparés du sang ou des composants sanguins.

13. Utilisation d'une membrane selon l'une des revendications 1 à 5 ou d'une membrane fabriquée d'après un procédé selon l'une des revendications 6 à 11 ou d'un polymère mixte qui se compose de 70 à 95 % en moles de motifs de monomère d'acrylonitrile et de N-vinylpyrrolidone avec une fraction molaire entre 5 % en moles et 30 % en moles, en particulier entre 15 % en moles et 30 % en moles, à titre de motifs de comonomère hydrosoluble non électrolytique, pour des applications médicales dans le corps, dans lesquelles elles sont confrontées aussi bien au sang qu'au tissu environnant.
